(19) Europäisches Patentamt / European Patent Office / Office européen des brevets

(11) **EP 1 275 414 B1**

(12) **FASCICULE DE BREVET EUROPEEN**

(45) Date de publication et mention
de la délivrance du brevet:
**11.04.2012   Bulletin 2012/15**

(51) Int Cl.:
***A61N 1/37*** *(2006.01)*

(21) Numéro de dépôt: **02291714.0**

(22) Date de dépôt: **09.07.2002**

(54) **Dispositif médical implantable actif pour le traitement des troubles du rythme cardiaque, comprenant des moyens de compensation du potentiel propre des amplificateurs des circuits de recueil**

Aktive implantierbare medizinische Vorrichtung zur Behandlung von Herzrhythmusstörungen, mit Mitteln zur Kompensation der selbstinduzierten Spannung der Verstärker der Detektionsschaltung

Active implantable medical device for treating heart rhythm disorders, having compensation means for the voltage self-induced by the detection circuit amplifiers

(84) Etats contractants désignés:
**AT BE BG CH CY CZ DE DK EE ES FI FR GB GR IE IT LI LU MC NL PT SE SK TR**

(30) Priorité: **11.07.2001   FR 0109183**

(43) Date de publication de la demande:
**15.01.2003   Bulletin 2003/03**

(73) Titulaire: **ELA MEDICAL**
**92541 Montrouge (FR)**

(72) Inventeur: **Casset, Cyril**
**75010 Paris (FR)**

(74) Mandataire: **Dupuis-Latour, Dominique et al**
**SEP Bardehle Pagenberg Dost Altenburg Geissler**
**10, boulevard Haussmann**
**75009 Paris (FR)**

(56) Documents cités:
**WO-A-98/22183        US-A- 4 674 509**
**US-A- 5 350 410      US-A- 6 144 881**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Tous ces dispositifs comportent des moyens de détection d'activité, c'est-à-dire de détection des dépolarisations spontanées du myocarde, ainsi que des moyens de stimulation de ce myocarde.

Il est important de pouvoir recueillir le signal résultant du rythme spontané du patient le plus tôt possible après la stimulation, afin de détecter le plus précocement possible une onde de dépolarisation révélatrice d'une activité spontanée des cellules myocardiques et permettre ainsi d'exécuter par exemple des algorithmes très précis de contrôle du rythme cardiaque donnant lieu à un comportement plus physiologique de la prothèse, ou encore permettre une réduction de la consommation en ne délivrant que des stimulations appropriées. Cette détection est également utilisée pour commander le fonctionnement de certains algorithmes tels que les algorithmes de repli ou de lissage, etc. D'autre part, la détection du rythme ventriculaire spontané, notamment l'analyse de sa stabilité, est dans certains défibrillateurs implantables un paramètre essentiel de déclenchement de la thérapie de choc.

La vérification de l'efficacité de la stimulation ou "test de capture" est de ce fait un paramètre essentiel pour maintenir l'implant dans sa plage de fonctionnement optimale.

Le test de capture se fait en mesurant le "potentiel évoqué", c'est-à-dire le potentiel de l'onde de dépolarisation induite par la stimulation de la cavité considérée.

Ce signal est toutefois biaisé par des perturbations liées au comportement des amplificateurs de recueil juste après la stimulation.

Un premier type de perturbation provient de l'écoulement des charges à l'interface électrode/myocarde. Pour l'éliminer, on prévoit une période dite "période réfractaire" pendant laquelle est opérée une déconnexion ou "blanking" des circuits de détection, typiquement pendant une durée de l'ordre de 13 ms (la stimulation ayant elle-même une durée de l'ordre de 1 ms maximum). Après cette période de blanking absolu, suit une période d'attente ou "écoute" de la réponse évoquée, d'une durée typique de l'or_ dre de 50 ms.

Le signal délivré par les circuits de détection pendant cette période d'écoute est cependant perturbé par un autre facteur, propre à l'amplificateur de détection, en raison d'un phénomène de "rebond" de ce dernier au moment où il est reconnecté aux bornes de l'électrode de détection à l'issue de la période de blanking.

Le signal délivré par l'amplificateur comprendra donc, s'il est présent, le potentiel évoqué résultant de la dépolarisation spontanée de la cavité, auquel sera superposé un potentiel de réponse propre à l'amplificateur, dont la présence et le niveau seront essentiellement indépendants de la présence ou de l'absence d'une dépolarisation ; ce potentiel propre à l'amplificateur, ci-après "potentiel de réponse" est donc susceptible de perturber le test de capture, tout particulièrement lorsque l'onde de dépolarisation présente une amplitude faible, comme cela est par exemple le cas d'une onde de dépolarisation auriculaire du fait de la petite masse musculaire de l'oreillette.

L'un des buts de la présente invention est de proposer un dispositif permettant de déterminer ce potentiel de réponse et d'en éliminer l'effet de masquage afin d'accroître la qualité du test de capture.

Un dispositif de ce type est décrit dans le document US 4 674 509 A, qui propose de générer des couples d'impulsions dont l'une est "capturante" (induit un potentiel évoqué) et l'autre ne l'est pas. La différence des potentiels de dépolarisation mesurés permet d'estimer l'artefact introduit par l'amplificateur.

Une application avantageuse - mais non limitative - de la présente invention est celui du test de capture "cycle à cycle", mis en oeuvre dans certains implants récents où l'on cherche à réduire la tension de stimulation jusqu'à un niveau proche du seuil d'entraînement (tension en-dessous de laquelle il n'y aura plus de capture). Une telle réduction de tension présente l'avantage de réduire l'énergie de stimulation appliquée, et donc la consommation correspondante du dispositif, augmentant ainsi corrélativement la durée de vie de l'implant. La contrepartie en est la nécessité de vérifier de façon très fréquente, typiquement à chaque cycle cardiaque, et non plus à intervalles périodiques (par exemple toutes les six heures), si la stimulation a été efficace ou non, de manière à réajuster la tension de stimulation réduite et/ou rebasculer sur une tension correspondant à une amplitude de sécurité.

La présente invention propose un dispositif du type général divulgué par le US 4 674 509 A précité, correspondant au préambule de la revendication 1, et comportant les éléments distinctifs énoncés dans la partie caractérisante de cette revendication.

Les sous-revendications visent des modes de mise en oeuvre particuliers, avantageux.

On va maintenant décrire un exemple de réalisation de l'invention, en référence à la figure unique annexée qui illustre schématiquement la variation, au cours du temps, de l'amplitude du signal délivré par l'amplificateur du circuit de mesure de l'activité cardiaque.

Comme on l'a indiqué plus haut, le but de la présente invention est la détermination d'un potentiel de réponse propre aux amplificateurs des circuits de recueil de l'activité cardiaque lors de la période de mesure suivant immédiatement la période de blanking faisant suite à la délivrance de l'impulsion de stimulation.

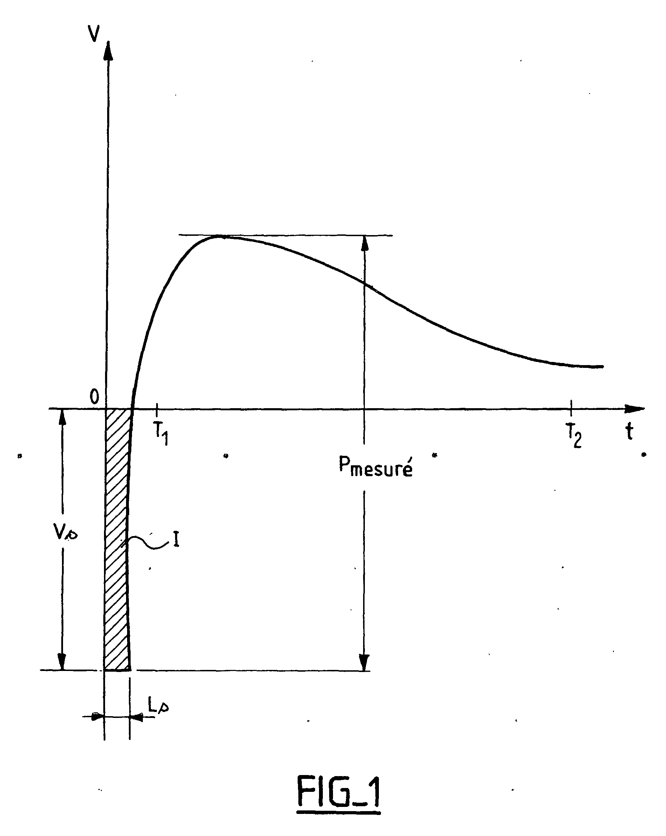

Le potentiel V délivré en sortie par l'amplificateur présente la forme caractéristique illustrée figure 1, qui donne l'évolution du signal de tension V en fonction du temps t (pour la commodité de la lecture, l'échelle des temps réels n'a pas été respectée).

Tout d'abord, le dispositif délivre une impulsion de stimulation I, d'amplitude (niveau de tension) $V_s$ et de largeur (durée d'impulsion) $L_s$. Ces deux paramètres $V_s$ et $L_s$ peuvent tous deux varier dans de larges proportions, en fonction des données de pilotage du stimulateur. L'amplitude $V_s$ est typiquement comprise entre 1,5 et 7,5 V, ajustable par pas de 0,5 V, et la largeur $L_s$ de l'impulsion est typiquement comprise entre 0,12 et 0,98 ms, ajustable par pas de 0,12 ms. L'énergie de stimulation est proportionnelle à la largeur $L_s$ et au carré de l'amplitude $V_s$ de l'impulsion.

Après chaque application d'une impulsion, il est prévu une période de blanking absolu, de durée typique $T_1 = 13$ ms, suivie par une période de mesure, typiquement de durée $T_2 - T_1 = 50$ ms pendant laquelle est attendue la réponse évoquée.

Pendant cette période, la tension délivrée en sortie de l'amplificateur évolue de la manière illustrée, avec un rebond de tension dû notamment à la décharge des capacités parasites de l'amplificateur au moment où celui-ci est rebranché à la fin de la période de blanking. Plus précisément, le dispositif détermine l'excursion de tension $P_{mesuré}$, qui est considérée comme la valeur de départ à partir de laquelle les calculs exposés par la suite seront effectués.

Le dispositif évalue ainsi une valeur d'amplitude comprenant le potentiel de dépolarisation (s'il est présent), auquel est superposé un potentiel de réponse propre à l'amplificateur ; ce potentiel de réponse est bien entendu un potentiel parasite venant masquer le potentiel de dépolarisation éventuellement présent, et il sera donc nécessaire d'en tenir compte pour évaluer la présence de ce potentiel de dépolarisation dans le signal global recueilli.

La difficulté qu'il y a à opérer cette discrimination est accrue par le fait que le potentiel de réponse propre à l'amplificateur (ci-après "potentiel de réponse") n'est pas une valeur constante, mais varie en fonction de l'impulsion de stimulation précédemment appliquée.

L'invention repose sur quatre hypothèses de base :

> 1°) Le signal délivré par l'amplificateur est la somme du potentiel de dépolarisation et du potentiel de réponse de l'amplificateur ;
> 2°) Le potentiel de réponse de l'amplificateur est sensiblement proportionnel à la tension de stimulation $V_s$ ;
> 3°) Le potentiel de réponse de l'amplificateur est proportionnel à la largeur $L_s$ de l'impulsion de stimulation ;
> 4°) Dès lors que l'énergie de l'impulsion de stimulation est suffisante pour dépolariser le myocarde, le potentiel évoqué est indépendant de l'énergie de stimulation : en d'autres termes, selon que l'on se situe au-dessous ou au-dessus du seuil d'entraînement, le potentiel évoqué est soit nul, soit de valeur sensiblement constante pour une cavité donnée ; le potentiel de dépolarisation ne varie donc pas de façon linéaire avec l'énergie de stimulation, à la différence du potentiel de réponse de l'amplificateur.

Si l'on désigne $P_{mesuré}$ le signal mesuré en sortie par l'amplificateur, $P_{coeur}$ le potentiel de dépolarisation et $P_{ampli}$ le potentiel de réponse propre à l'amplificateur, il vient :

$$P_{mesuré} = P_{cœur} + P_{ampli}$$

et

$$P_{ampli} = K.V_s.L_s$$

Sur ces bases, il est possible de définir une méthode de mesure du potentiel de dépolarisation, indépendamment du potentiel de réponse de l'amplificateur, afin de contrôler la présence ou l'absence d'une capture.

À cet effet, le dispositif est piloté de manière à appliquer deux impulsions de stimulation présentant des largeurs et/ou des niveaux de tension différents, mais dont on est certain que ces impulsions seront toutes capturantes, c'est-à-dire provoqueront avec certitude une dépolarisation.

En d'autres termes, on définit deux couples de valeurs $\{V_i, L_i\}$ et $\{V_j, L_j\}$ assurant la capture du myocarde.

Pour chacune de ces impulsions on mesure le potentiel total délivré par l'amplificateur, soit :

$$P_{mesuré}(i) = P_{cœur} + P_{ampli}(i)$$

et

$$P_{mesuré}(j) = P_{cœur} + P_{ampli}(j).$$

ou encore :

$$P_{mesuré}(i) = P_{cœur} + K.V_i.L_i$$

et

$$P_{mesuré}(j) = P_{cœur} + K.V_j.L_j.$$

On a ainsi constitué un système de deux équations à deux inconnues, $P_{coeur}$ et K, qui permet par le calcul d'extraire la valeur de ces deux paramètres.

Une fois que l'on a déterminé K, il est possible, pour toute stimulation ultérieure, d'éliminer dans le signal délivré le potentiel de réponse propre à l'amplificateur et donc d'établir avec un excellent degré de certitude la présence ou l'absence du potentiel de dépolarisation consécutif à l'impulsion.

Les relations liant le potentiel de dépolarisation et le potentiel mesuré ayant été ainsi établies de façon univoque, il est possible d'évaluer par extrapolation la réponse que l'on devrait obtenir pour d'autres tensions, largeurs ou énergies de stimulation, et contrôler ainsi la présence de $P_{coeur}$, donc déterminer s'il y a eu capture ou non.

## Revendications

**1.** Un dispositif médical implantable actif, notamment un stimulateur cardiaque, défibrillateur, cardioverteur ou dispositif multisite, comprenant :

- des moyens de stimulation, aptes à délivrer à au moins une cavité cardiaque des couples d'impulsions électriques de niveaux de tension et de largeurs prédéterminées, dont les impulsions sont deux impulsions de stimulation (i) aptes à provoquer chacune une dé- polarisation et (ii) dont les niveaux de tension et/ou les largeurs d'im- pulsion sont différents, et

- des moyens de recueil d'activité cardiaque, comportant au moins un amplificateur de signaux recueillis par une électrode intracardiaque, délivrant en sortie une valeur de potentiel mesuré ($P_{mesuré}$) consécutif à l'application d'une impulsion (I) par les moyens de stimulation,

- des moyens de détermination d'un potentiel de réponse propre audit amplificateur, indépendamment de la présence ou de l'absence d'une dépolarisation myocardique,

dispositif **caractérisé** :

- et en ce que les moyens de détermination du potentiel de réponse comprennent des moyens pour déterminer, à partir des potentiels mesurés respectifs recueillis correspondant à ces deux impulsions, une constante et un coefficient de proportionnalité d'une relation linéaire liant le niveau du potentiel mesuré au produit de la largeur par le niveau de tension de l'impulsion.

**2.** Le dispositif médical implantable de la revendication 1, comprenant en outre:

- des moyens compensateurs aptes à soustraire, de la valeur de potentiel mesuré ($P_{mesuré}$) consécutif à l'application d'une impulsion (I) par les moyens de stimulation, la valeur de potentiel de réponse propre à l'amplificateur correspondant à la largeur ($L_s$) et au niveau de tension ($V_s$) de ladite impulsion.

**3.** Le dispositif médical implantable de la revendication 2, comprenant en outre :

- des moyens de test de capture aptes, après délivrance d'une impulsion par les moyens de stimulation, à comparer la valeur de potentiel délivrée en sortie des moyens compensateurs à un seuil prédéterminé et, si la valeur est supérieure au seuil, décider la présence d'un potentiel évoqué résultant de la dépolarisation myocardique.

## Claims

**1.** Implantable active medical device, especially a pacemaker, a defibrillator, a cardioverter-defibrillator or a multisite pacing device, comprising:

- stimulating means, able to deliver, to at least one heart chamber, pairs of electrical impulses of preset durations and voltage levels, the two stimulation impulses of which (i) are each able to cause depolarization and (ii) each have different voltage levels and/or durations;

- means for capturing cardiac activity, comprising at least one amplifier for amplifying signals captured by an intracardiac electrode, said amplifier delivering as output a measured potential ($P_{measured}$) following application of an impulse (I) by the stimulating means; and

- means for determining a response potential specific to said amplifier, independently of the presence or absence of a myocardial depolarization,

which device is **characterized in that**

- the means for determining the response potential comprise means for determining, from the respective measured potentials captured that correspond to these two impulses, a constant and a coefficient of proportionality for a linear relationship between the level of the measured potential and the product of the duration and the voltage level of the impulse.

**2.** Implantable medical device according to Claim 1, furthermore comprising:

- compensating means able to subtract the response potential specific to the amplifier, corresponding to the duration ($L_s$) and the voltage level ($V_s$) of said impulse, from the measured potential ($P_{measured}$) delivered following application of an impulse (I) by the stimulating means.

3. Implantable medical device according to Claim 2, furthermore comprising:

- capture-testing means able to compare, after an impulse has been delivered via the stimulating means, the potential delivered as output from the compensating means with a preset threshold and, if the potential is above this threshold, able to determine that an incited potential resulting from myocardial depolarization is present.

**Patentansprüche**

1. Aktive implantierbare medizinische Vorrichtung, insbesondere Herzstimulator, Defibrillator, Kardioverter oder Mehrstellen-Vorrichtung, die Folgendes umfasst:

- Stimulationsmittel, die an wenigstens einen Herzhohlraum Paare elektrischer Impulse mit vorgegebenen Spannungspegeln und Breiten verabreichen können, wobei die Impulse zwei Stimulationsimpulse sind, die (i) jeweils eine Depolarisation hervorrufen können und (ii) deren Spannungspegel und/oder Impulsbreiten verschieden sind, und
- Mittel zum Erfassen einer Herzaktivität, die wenigstens einen Verstärker für Signale, die von einer intrakardialen Elektrode erfasst werden, enthalten und nach dem Anlegen eines Impulses (I) durch die Stimulationsmittel am Ausgang einen gemessenen Potentialwert ($P_{mesure}$) ausgeben, und
- Mittel zum Bestimmen eines Antwortpotentials des Verstärkers unabhängig vom Vorhandensein oder Fehlen einer myokardischen Depolarisation,

wobei die Vorrichtung **dadurch gekennzeichnet ist, dass**:

- die Mittel zum Bestimmen des Antwortpotentials Mittel umfassen, um anhand erfasster jeweiliger gemessener Potentiale, die diesen zwei Impulsen entsprechen, eine Konstante und einen Proportionalitätskoeffizienten einer linearen Beziehung, die den gemessenen Potentialpegel mit dem Produkt aus der Breite und dem Spannungspegel des Impulses in Beziehung setzt, zu bestimmen.

2. Implantierbare medizinische Vorrichtung nach Anspruch 1, die außerdem Folgendes umfasst:

- Kompensationsmittel, die von dem Wert des gemessenen Potentials ($P_{mesure}$) nach dem Anlegen eines Impulses (I) durch die Stimulationsmittel den Wert des Antwortpotentials des Verstärkers, der der Breite ($L_s$) und dem Spannungspegel ($V_s$) des Impulses entspricht, subtrahieren können.

3. Implantierbare medizinische Vorrichtung nach Anspruch 2, die außerdem Folgendes umfasst:

- Aufnahmetestmittel, die nach der Verabreichung eines Impulses durch die Stimulationsmittel den Wert des ausgegebenen Potentials am Ausgang der Kompensationsmittel mit einem vorgegebenen Schwellenwert vergleichen können und, falls der Wert größer als der Schwellenwert ist, entscheiden können, dass ein hervorgerufenes Potential vorhanden ist, das sich aus der myokardischen Depolarisation ergibt.

FIG_1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4674509 A